# EUROPEAN PATENT APPLICATION

(11) **EP 4 494 562 A1**
(43) Date of publication of application: **22.01.2025**
(21) Application number: 23206251.3
(22) Date of filing: 26.10.2023
(51) Int. Cl.: A61B 5/374

(54) **A COMPUTER-IMPLEMENTED METHOD FOR DETERMINING A SLEEP RESTORATION INDEX INDICATIVE OF A CHANGE OF SLOPE OF SLOW WAVES**

(30) Priority: 18.07.2023 EP 23186021
(71) Applicant: Universität Zürich, 8006 Zürich (CH)
(72) Inventor: HUBER, Reto, 8032 Zürich (CH); SKORUCAK, Jelena, 8032 Zürich (CH)
(74) Representative: Schulz Junghans Patentanwälte PartGmbB

(57) **Abstract**

The invention relates to a computer-implemented method for determining a sleep restoration index indicative of a change of slope of slow waves during a non-REM sleep phase. The method employs a first neural networks for determining and filtering out epileptic spikes in the EEG data and a second neural network for identifying and labeling sleep stages. Combining the information generated by these networks, slow waves are identified and a change of slope of the slow waves is determined.

The invention also relates to an EEG system and a computer program configured to executes the method.

## Description

The invention relates to a computer-implemented method for determining a sleep restoration index, a system to execute the method and a computer program configured to execute the method.

Sleep plays a vital role in many physiological functions, such as memory consolidation, synaptic plasticity, and brain metabolic waste clearance. These functions are presumably underlying the fact that sleep is closely related to behavioral performance: insufficient sleep restoration during the night will lead to behavioral deficits during the following day. Importantly, only sleep can revert these performance deficits. Non-rapid eye-movement, non-REM or NREM, sleep including deep sleep is essential for the restorative functions of sleep. Although the mechanisms underlying the restorative function of sleep are still not fully understood, according to the synaptic homeostasis hypothesis (SHY), sleep plays a role in synaptic plasticity: Synaptic strength needs to be reduced during sleep to renormalize the experience-dependent increases that occur during wakefulness. Human, animal and modelling data shows that the slope of slow-waves is the most direct EEG measure for synaptic strength. As expected based on the predictions of SHY, numerous studies have shown overnight decrease in the slope of slow waves, which is reflective of an overnight reduction in synaptic strength. In childhood epilepsies with a spike-wave activation during sleep, epileptic spikes invade NREM sleep. For decades it has been postulated that these spikes are the origin of cognitive and behavioral deficits these patients suffer from. These impairments can be as dramatic as loss of language abilities or a global developmental regression. Recent studies provide compelling evidence that the overnight decrease in slope is critically dampened in childhood epilepsies with spike-wave activation during sleep. Interestingly, these same patient group shows significant deficits in sleep-dependent memory consolidation. It is tempting to assume that impaired consolidation of newly learned information might lead with time to the patient's behavioral and cognitive deficits. One of the key-limiting factors for this research topic is the fact that so far sleep and epilepsy in pediatric patients has been investigated in only small-size datasets. Investigating the effect of spikes on sleep in large-scale datasets is only feasible by implementing an automated approach. Furthermore, if cognitive impairment in epilepsy can be linked to both compromised overnight decrease in slow wave slope and spikes in sleep, other disorders may also benefit from this approach.

The identification of specific sleep- and/or spike-related deficits in these disorders can help to disentangle their underlying pathomechanisms. Ultimately, an automated computer-implemented method for sleep and spike detection may be incorporated into clinical diagnostics providing new and easily accessible information to clinicians.

An object of the present invention is to provide such an automated method for unsupervised, automatic processing, evaluating and labeling of EEG data extending over the whole sleep duration of a patient. A task no human expert may be capable to execute within a reasonable amount of time.

The object is achieved by the method having the features of claim 1.

Advantageous embodiments are described in the dependent claims.

According to a first aspect of the invention, a method, particularly a computer-implemented method for determining a sleep restoration index indicative of a change of slope of slow waves during a non-REM sleep phase, particularly in patients in the age between 4 and 26 years, comprises the steps of:
a. Acquiring on a computer EEG data recorded from a patient, the EEG data comprising information on a plurality of EEG channels, particularly wherein each EEG channel reflects an electrical activity, particularly wherein the EEG data is provided in form of an electrogram recorded of the patient during sleep,
b. Providing a first data set comprising information on one or more EEG channels of the plurality of EEG channels to an epileptic spike detector program module comprising a first artificial neural network configured, particularly trained to detect and assign periods of spikes, particularly during non-REM sleep stages, in the at least one EEG channel comprised by the first data set,
c. Providing a second data set comprising information on one or more EEG channels of the plurality of EEG channels to a sleep detector program module comprising a second artificial neural network configured, particularly trained to detect and assign sleep stages to the one or more EEG channels comprised by the second data set, wherein the detected sleep stages are selected from the group consisting of periods of non-REM sleep stages 2 and 3, and periods of other sleep stages,
d. Providing a third data set comprising information on one or more EEG channels of the plurality of EEG to a slow wave detector program module configured to detect and assign slow waves in the one or more EEG channels comprised by the third data set, wherein periods assigned to spikes as well periods assigned to the other sleep stages are omitted for slow wave detection in the third data set,
e. Determining for at least a first episode of a period assigned to non-REM sleep stages 2 and 3 and a last episode of the period assigned to non-REM sleep stages 2 and 3 from detected slow waves, particularly for each detected slow wave a slope value,
f. Determining a change of slope values between the first and the last episode, particularly a change of an average slope value determined from an average of slope values of the first and an average of slope values of the last episode, particularly wherein the change of slope is expressed in percent,
g. Determining and particularly outputting a sleep restoration index indicative for the change of the slope values.

The method allows for an automatic, robust and efficient determination of the change of slope values over long stretches of recording of EEG data. This takes usually the experienced analysis of experts, which is tedious and time-consuming.

The EEG data typically comprises information on a plurality of EEG-channels, for each processing step a. to d. at least one EEG channel is processed by the corresponding module. While it is possible that the first, second and/or the third data set comprise information on the same one or more EEG channels it is also conceivable that each data set of the first, second and/or third data set comprises information on different EEG channels.

Further, the data sets may comprise information on a plurality of EEG-channels each, or even of all recorded EEG-channels.

An EEG-channel typically comprises information on a temporal evolution of an EEG sensor signal, such as an electrode signal, comprising voltages recorded at a specific region at the head of the patient.

As each EEG-channel is assigned to a specific EEG sensor it is possible to assign the EEG-channel to a specific region of the head of the patient.

The EEG data may be acquired from a computer, a memory storage, or an EEG measurement system.

The term "electrogram" particularly refers to a set of EEG channels.

The term "indicative" particularly refers to one or more selected from of the group consisting of: derived of, associated to , dependent of, correlated to.

The epileptic spike detector program module may be embodied as a computer program that is executed during the execution of the method, or in form of a hardware module that is configured to execute the first artificial neural network.

The spikes to be detected and assigned by the spike detector program module are for example spikes that occur in the context of epileptic seizures or pre-epileptic stages.

These spikes, if left unattended for the determination of the sleep restoration index, in particular to the change of slope of the slow waves, could distort the automatic evaluation of the sleep restoration index. Therefore, the synergetic cooperation of the first and the second artificial neural network allows for the robust and reliable determination of the sleep restoration index.

While the first artificial neural network is trained to detect the spikes in the EEG data, and particularly to also determine a focus of the spikes, i.e. the electrodes that are closest to the origin of the spikes, the second artificial neural network is trained to determine the sleep stages.

With regard to the sleep detector program module the same holds true as for the spike detector program module, namely that it may be embodied as a computer program or in form of a hardware module configured to execute the second artificial neural network.

Having identified and assigned the sleep stages and periods of spikes, only then it becomes possible to determine with the slow wave detector program module the slow waves in the EEG data in one or more EEG channels.

The other sleep stages that may be less interesting for the determination of the restoration index are for example REM, non-REM stage 1, wake. The sleep stage detector program module may be further configured to also detect and assign said other sleep stages to REM, non-RAM stage 1 and wake. This may be useful for further evaluation the EEG data.

Having identified that slow waves it further becomes possible to determine a slope as well as a change of the slope of the slow waves in subsequent steps.

The invention sets out to determine the slope in at least a first episode and a last episode, such that a change of slope may be determined at least with respect to the first and the last episode.

The change of the slope is indicative of a restorative as well as a regenerative effect during sleep of the patient.

The restoration index may be a single value, a set of values as well as values as a function of time.

Optionally, the sleep restoration index and/or the change of slope of the slow waves may be put out on a display or another user interface that method might control.

Slope wave values may not be determined or omitted in further evaluation in case the detect slow wave is detected in a predefined proximity to a detected spike, particularly wherein the proximity is a time interval having a duration in the range of 0s to 4s, particularly 0.2s to 2s, more particularly, 0.3s to 1s, more particularly 0.4s to 0.6s.

According to another embodiment of the invention, from the periods of spikes in the at least one EEG channel comprised by the first data set, a spike wave index is determined, wherein the spike wave index is indicative of a spike rate during the periods of spikes, particularly wherein the spike wave index equals a number of spikes per time interval, e.g. 10s, particularly wherein the spike wave index is expressed in percent.

The spike wave index may be determined as follows:
The period of spikes is subdivided in one or more intervals of constant duration, e.g. 10 s intervals. Each interval in turn is subdivided in subintervals, e.g. 1s intervals. For each interval and its subintervals, it is determined whether there is one or more spike occurring in the subinterval. In case one or more spikes are detected in a subinterval, a value of "1" is assigned to said subinterval. In case no spike occurs in the subinterval a value of "0" is assigned to said subinterval. For each interval the sum of all values of the subintervals comprised by the interval is calculated and normalized to, e.g. divided by the number of subintervals in the interval.

The spike wave index allows for quantification of an intensity of the epileptic episode during sleep and thus may be taken into account with the sleep restoration index as an additional information.

Optionally, the spike wave index may be put out on the display or another user interface together with the sleep restoration index.

According to another embodiment of the invention, the spike detector program module determines the spike wave index for a multitude of EEG channels or each EEG channel of the EEG data, wherein based on an associated position of the EEG channels on the head of the patient, a spike wave index map of the head of the patient is generated and particularly displayed, wherein said spike wave index map comprises information on the spatial distribution of the spike wave indices, particularly the spatial distribution of an intensity of the spike wave index.

This allows for localizing epileptic foci during sleep of the patient. The map might have a form of a projection to a predefined head-shape template. Assigning the spike wave index to the correct location on said map may be based on the position of the EEG sensor.

The spike wave index may be displayed on said map using a color map indicative of the intensity / magnitude of the spike wave index.

According to another embodiment of the invention, the sleep detector program module detects and assigns the sleep stage for a multitude of EEG channels or each EEG channel of the EEG data, and the sleep restoration index is determined for the EEG channel, wherein based on an associated position of the EEG channel of the EEG data on the head of the patient, a sleep restoration index map of the head of the patient is generated and displayed, wherein said sleep restoration index map comprises information on the spatial distribution of the change of slope of slow waves between the first and the last episode.

This embodiment allows similarly to the previous embodiment to gain an understanding of spatial variations of the change of slope of the slow waves. The map may be a pre-defined head template map, to which the result for each EEG-channel is assigned to accordingly.

According to another embodiment of the invention, a correlation value between the change of slope and the spike wave index is determined, particularly wherein the correlation value is displayed, particularly together with the sleep restoration index or any of the maps generated.

The correlation value may be indicative of epileptic episodes affecting sleep quality of the patient, allowing to determine efficiency of a treatment e.g. with a drug for supressing epileptic spikes or alleviating impact of epileptic seizures.

According to another embodiment of the invention, the detected spikes are removed from the second data set, particularly subtracted from the EEG channels in which they have been detected, such that the EEG channels processed by the sleep detector program module are devoid of spikes detected by the spike detector program module, and/or wherein the detected spikes in the periods of spikes are removed from the third data set, such that the EEG channels processed by the slow wave detector program module are devoid of spikes detected by the spike detector program module.

This allows for downstream processing without distortion of the spikes being present in the EEG-channel(s).

According to another embodiment of the invention, the second data set is processed particularly by the sleep detector program module to obtain a temporal power spectrum of at least three EEG channels, wherein said power spectrum is provided to the second artificial neural network as an input feature.

This allows for a robust detection of the sleep stages.

According to another embodiment of the invention, a recording time of the electrogram comprised by the EEG data is longer than 5 minutes, particularly longer than 0,5 hours, particularly longer than 1 hours, more particularly longer than 2 to 4 hours, having a resolution of at least 128 Hz.

This embodiment allows for processing and analysing long stretches of EEG data that would be too time-consuming to be analysed by an expert.

Furthermore, short recording times may sufficient to determine the spike wave index map.

Also, the longer the recording time the more likely it is to observe the relevant sleep stages.

According to another embodiment of the invention, recording the EEG data and processing the EEG data according to at least the steps a. to g. is executed simultaneously.

This embodiment allows for real-time or quasi real-time evaluation of the EEG data as far as possible to at least provide preliminary results on sleep stages and periods of spikes. Determination of the change of slope of slow waves may be done once sufficient information is obtained, e.g. when the sleep has been long enough to be considered for such an evaluation.

According to another embodiment of the invention, the EEG data is transmitted via a data network to a cloud server comprising the computer to execute at least the steps a. to d..

This allows for locating necessary computational infrastructure apart from the place of measurement, which in turn allows for many sleep-analysing labs and even-home recording of the EEG data to benefit from the method according to the invention. Maintenance of the software executing the method may also be executed on a single system rather than updating a plethora of computers.

According to another embodiment of the invention, the method is executed repeatedly, particularly daily or weekly, on the same patient allowing for monitoring of the sleep of the patient and particularly to detect an effect of a drug administered to the patient, more particularly to adjust a dosage of a drug administered to the patient depending on the sleep restoration index.

According to another embodiment of the invention, the first artificial neural network comprises or is recurrent neural network, particularly a long short-term memory recurrent neural network and/or wherein the second artificial neural network comprises or is recurrent neural network, particularly a long short-term memory recurrent neural network.

Recurrent neural networks are specifically well-suited to be applied to sequential data, such as EEG data.

Long short-term memory recurrent neural networks allow for improved handling of long-term effects in the EEG data, which particularly for determining the sleep stages is an advantageous feature, for batch processing.

According to another embodiment of the invention, at least some or all EEG channels are annotated with the detected sleep stages and with the detected periods of spikes, particularly with the corresponding spike wave index.

This allows for post-recording inspection by an expert. The annotations are helpful in order to quickly identify periods of spikes in different sleep stages as well periods which were used for slow wave detection.

According to a second aspect of the invention, an EEG-system comprises a wearable EEG assembly with EEG sensors, such as a net or a headcover, such as a cap or helmet, with EEG sensors, such as electrodes, configured to record EEG data. The EEG system further comprises a computational system, comprising a processor. The computational system is connected, e.g. wirelessy by means of an electromagnetic wave transmission module, configured e.g. as a Bluetooth module, with the wearable EEG assembly to receive EEG data recorded by the assembly, wherein the computational system is configured to executed the method according to the first aspect of the invention.

The computational system may comprise one or more computers located at the EEG assembly or at different locations, for distributed task execution. The computational system may be located at a server receiving the EEG data from the transmission module, e.g. via a data network.

Definitions and features of the method according to the first aspect may be used to interpret and limit the invention according to the second aspect and vice versa.

The EEG system allows for recording EEG data at home for the patient.

According to a third aspect of the invention, a computer program comprises computer program code that when executed on a computer or on the computational system, executes the method according to the first aspect.

The term computer program comprises the notion of a computer program product stored on a non-transitory storage medium that may be comprised by the computer or the computational system.

According to a fourth independent aspect of the invention, a method, particularly a computer-implemented method for determining a spike wave index map, particularly in patients in the age between 4 and 26 years, comprises the steps of:
a. Acquiring on a computer EEG data recorded from a patient, the EEG data comprising information on a plurality of EEG channels, particularly wherein each EEG channel reflects an electrical activity, particularly wherein the EEG data is provided in form of an electrogram recorded of the patient during sleep,
b. Providing a first data set comprising information on one or more EEG channels of the plurality of EEG channels to an epileptic spike detector program module comprising a first artificial neural network configured, particularly trained to detect and assign periods of spikes, particularly during non-REM sleep stages, in the at least one EEG channel comprised by the first data set,
c. determining with the spike detector program module a spike wave index for a multitude of EEG channels or each EEG channel of the EEG data, wherein based on an associated position of the EEG channels on the head of the patient, a spike wave index map of the head of the patient is generated and particularly displayed, wherein said spike wave index map comprises information on the spatial distribution of the spike wave indices, particularly the spatial distribution of an intensity of the spike wave index.

This allows for localizing epileptic foci during sleep of the patient. The map might have a form of a projection to a predefined head-shape template. Assigning the spike wave index to the correct location on said map may be based on the position of the EEG sensor.

The spike wave index may be displayed on said map using a color map indicative of the intensity / magnitude of the spike wave index.

The spike wave index may be determined from the periods of spikes in the multitude of EEG channels comprised by the first data set, wherein the spike wave index is indicative of a spike rate during the periods of spikes, particularly wherein the spike wave index equals a number of spikes per time interval, e.g. 10s, particularly wherein the spike wave index is expressed in percent.

The definitions, embodiments, and/or features of the invention according to the first to third aspect may be applied to further define or interpret the fourth independent aspect of the invention.

Particularly with regard to the spike detector program module, it is noted that the functionality and structure may be identical as laid out for the spike detector program module according to the first aspect of the invention.

Furthermore, the system according to the second aspect of the invention may be configured to execute the method of the fourth independent aspect, particularly instead or in combination with the method according to the first aspect.

A fifth aspect relates to a computer program comprising computer program code that when executed on a computer or on the computational system, executes the method according to the fourth aspect.

The term computer program comprises the notion of a computer program product stored on a non-transitory storage medium that may be comprised by the computer or the computational system.

Particularly, exemplary embodiments are described below in conjunction with the Figures. The Figures are appended to the claims and are accompanied by text explaining individual features of the shown embodiments and aspects of the present invention. Each individual feature shown in the Figures and/or mentioned in said text of the Figures may be incorporated (also in an isolated fashion) into a claim relating to the device according to the present invention.
- Fig. 1: shows EEG data of a sleeping patient and the evaluation results of experts and the method according to the invention;
- Fig. 2: shows a portion of EEG data with detected slopes of slow waves;
- Fig. 3: shows a statistical evaluation of the change of slope of the slow waves;
- Fig. 4: shows a flow diagram of the signal processing according to one embodiment of the invention;
- Fig. 5: shows the percentage of spike wave index and the percentage of the change of slope mapped to regions of the head;
- Fig. 6: shows schematically EEG data comprising a plurality of EEG channels

### Development of the method

In a first step, the program modules for sleep stage detection and epileptic spike detection are developed based on a small-scale EEG dataset comprising EEG data that is scored by clinical neurophysiology experts. In a next step, the program modules were then applied to a large-scale EEG dataset, not scored by experts.

The small-scale data set was obtained by pooling EEG data from several previous studies including 39 pediatric patients with epilepsies with a spike-wave activation during sleep was used for the development and testing of the automatic sleep stage detection and spike detection. Sleep was scored by an expert in 20-s epochs during the entire recording night according to AASM criteria. Spikes were marked by an expert (SS) in a 10-min EEG data segment for each patient, starting after a first sleep stage 2. Further, in order to estimate inter-rater variability, 1-min EEG data segments were marked by two experts (SS and BS) in 20 acquired EEG data.

The epileptic spike detector program module and the sleep detector program module were developed in this dataset and then applied to the large-scale dataset.

The large-scale dataset contains 39'179 EEG recordings performed at the University Children's 101 Hospital Zurich in 1994-2019 and was available for analysis. These EEG recordings did not contain systematic annotations of sleep stages or periods of spikes. In the first step, recordings with duration ≥ 4 h (candidate recordings) were considered, since these were more likely to contain different sleep stages. Of 1'553 candidate recordings, 695 recordings included sleep recordings of ≥ 4h. Other than sleep duration, no other exclusion criteria were applied. Random exclusion of repeat recordings from the same patient, resulted in 413 EEG recordings (each from a single patient) included for slope of slow waves analysis. Exclusion of patients with age and diagnosis which were not electronically accessible in an automated fashion resulted in 357 patients included for age analysis and 296 for diagnosis analysis.

### EEG recordings and data pre-processing

EEG sensors, in form of EEG electrodes were placed according to the international 10-20 system. Recordings were executed at a 128 Hz or 256 Hz sampling rate by the Deltamed^{®} (Paris, France) EEG system. EEG data were exported to the European Data Format (EDF) and further processed in MATLAB R2020a (The Math Works Inc., Natick, MA). The following filters were applied: a bandpass 0.3 Hz - 40 Hz, and a notch filter at 50 Hz. EEG data were re-referenced to the contralateral mastoids, and down-sampled to 128 Hz, where applicable.

### Classifiers

Two classifiers were developed: 1) the epileptic spike detector program module, and the sleep detector program module. Both classifiers comprised a long short-term memory (LSTM) recurrent neural network. Recurrent neural networks advantageously perform on temporal structure of data and therefore exhibit a good performance for EEG data. The small-scale dataset, used for the classifier development, was divided into a training (27 patients), validation (6 123 patients), and testing dataset (6 patients). The classifier architecture for each program module was optimized by manual tuning of 1) the number of neurons in the hidden layer, the number of hidden layers, and the inclusion and probability of dropout layers. The best performing classifier architecture was then used for the test set applied to the large-scale dataset.

### The epileptic spike detector program module

The epileptic spike detector program module was developed based on expert scoring and raw data. The structure of the epileptic spike detector program module embodied as an LSTM was as follows: an input layer (1 neuron), two LSTM layers (130 neurons each) each followed by a dropout layer (dropout probability 0.1), followed by a fully connected layer (2 neurons), a softmax layer, and a classification output layer. Six training epochs were applied, i.e., the entire training data were passed through the epileptic spike detector program module six times. The adaptive moment estimation optimization algorithm (Adam) was used to update epileptic spike detector program module, particularly the network weights during training. The input of the epileptic spike detector program module consisted of a moving time window of 1/16 s (16 samples; step 125 ms).

### Sleep detector program module

Since overnight change in slope of slow waves depends on NREM sleep stages 2 and 3, automated scoring to the detection of NREM sleep was limited to stages 2 and 3. Hence, only two classes were identified by the sleep detector program module: NREM sleep stages 2 or 3, and all other stages (REM sleep, wake and NREM sleep stage 1). The sleep detector program module was developed based on the expert sleep scoring and engineered features. The inventors used feature engineering to extract quantifiable properties of the sleep EEG, calculated from power spectral analysis of three EEG channels comprised in the EEG data: F3-M2, C3-M2, and O1-M2 as: power in alpha (8 Hz - 12 Hz), sigma (i.e., spindle range; 12 Hz - 16 Hz), delta (i.e. slow wave activity, SWA, 0.75 Hz - 4.5 Hz), and artifact range (30 Hz - 40 Hz). Spectral analysis of EEG channels was performed on consecutive 20 s epochs (FFT, Tukey window [r = 0.5], average of five 4-s epochs; matched with sleep stages), resulting in a 0.25 Hz frequency resolution. As epileptic spikes may distort the power spectra, spectral analysis was performed after removing epileptic spikes from the EEG data, as detected by the epileptic spike detector program module. The sleep detector program module has been embodied as a (second) LTSM recurrent neural network (RNN), wherein the structure of the (second) LSTM-RNN has been selected as follows: an input layer (12 neurons), two LSTM layers (64 neurons each), each followed by a dropout layer (dropout probability 0.1), followed by a fully connected layer (two neurons), a softmax layer, and a classification output layer. Twenty training epochs were applied. The input of the LSTM consisted of a moving time window of 11 sleep epochs 153 (220 s; step: one sleep epoch or 20 s).

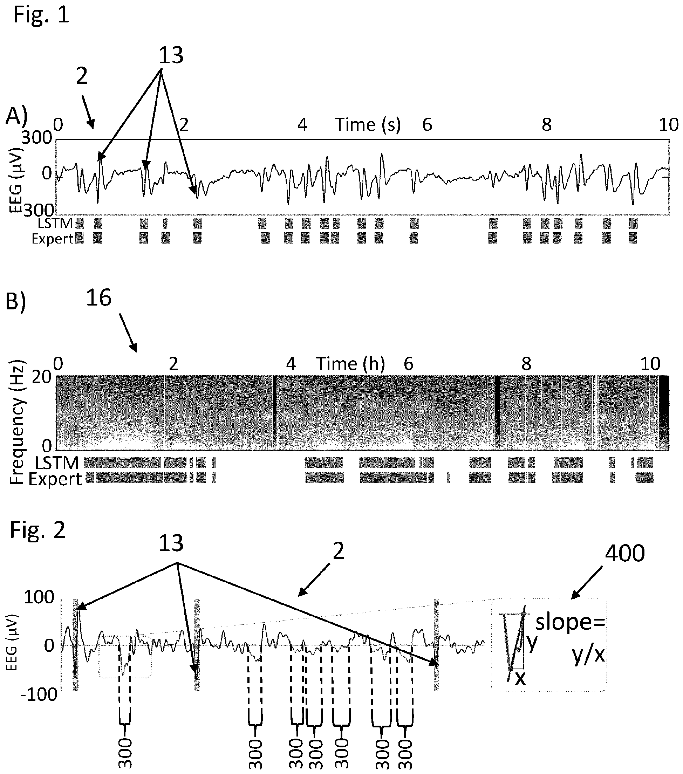

The slope of slow waves was calculated for the first and last hour of NREM sleep, as illustrated in Fig. 2. The inventors investigated whether they could replicate previous findings in healthy children. The correlation between the age of the patients and the slow wave slopes in the first, the last hour of NREM sleep, and the overnight change in slope was assessed. The slopes both in the first and last hour of sleep were negatively correlated with age, and overnight change in slope did not show correlation with age.

Fig. 2) shows a 10-s EEG channel trace with epileptic spikes in boxed areas 13, and slow waves in some regions (indicated by 300), as identified by the program modules. Note that slow waves occurring 0.5 s after the spike were not considered for analysis. Also, only slow waves that were matched in the amplitude matching procedure between the first and last hour of NREM sleep were further analyzed. The slope was calculated as the ascending slope of slow waves from the negative peak to the zero crossing (cf. magnified box 400)

### Assessment of classifier performance

The performance of the classifiers, i.e. the sleep detector program module and the epileptic spike detector program module, was assessed by determining specificity, sensitivity, precision, accuracy, and the Cohen's kappa coefficient. The inventors focused on Cohen's kappa coefficient, as it is a robust measure, accounting for the possibility of the agreement occurring by chance. The inventors interpreted the performance results for Cohen's kappa using Landis and Koch levels: <0.00-poor; 0.00-0.20-slight; 0.21-0.40-fair; 0.41-0.60-moderate; 0.61- 0.80-substantial; 0.81-1.00-almost perfect identification. Overall performance measures across all patients (pooled data) and mean values across patients were reported.

As exemplified in the EEG channel including epileptic activity (Fig. 1A) there was a good overlap between expert and spike detection with the epileptic spike detector program module. The time-frequency plot of an exemplary night (Fig. 1B) shows a similarly good overlap between the expert and automated NREM sleep stages 2 and 3 classification executed by the sleep detector program module. When quantifying these overlaps, both the automatic detection of epileptic spikes and the detection of sleep stages showed good performance, with a Cohen's kappa coefficient of 0.72 and 0.80, and an accuracy of 92.3% and 90.3% respectively. On a validation dataset, Cohen's kappa coefficient was 0.73 for spike detection and 0.71 for sleep detection, while accuracy was 93.7% and 86.4%. Cohen's kappa of inter-scorer reliability calculated in 1-min EEG segments of 20 patients was 0.71 for the spike detection, indicating similar agreement between two experts as between one expert and the program modules performance.

Fig. 1A shows spike detection ten-second EEG trace with epileptic activity. Fig. 1B shows sleep detection (spectrogram of a 10-h night EEG recording with frequency at the y-axis and time at x-axis, and darker gray values representing higher power values of a patient with spike-wave epilepsy. Expert scoring is depicted in dark gray (Expert) and automatic detection with the spike detector program module in lighter gray (LSTM), below the respective graph.

### Assessment of inter-scorer variability for spike detection

Out of 39 patient recordings, 20 were scored independently by two different experts. These records were randomly selected. One minute of EEG data was scored for epileptic spikes. Performance measures were calculated in the same way as for the classifiers.

### Determination of the sleep restoration index

The sleep restoration index was estimated by calculating overnight change in the slope of slow waves during NREM sleep.

For detecting the slow waves first, the signal was band-pass filtered (0.5 Hz - 4.0 Hz, stopband 0.1 and 10 Hz, Chebyshev Type II filter). Negative deflections between two zero-crossings were identified as slow-waves if they were separated by 0.25-1.0 s. The ascending slope of slow waves was determined by calculating the amplitude divided by the time from the most negative peak to the second zero-crossing (Fig. 2). As it is unknown if slow waves directly associated with epileptic spikes, so called spike-wave complexes, contribute to sleep homeostasis, all slow waves occurring in a window of 0.5 s after the epileptic spike were excluded from further analyses. The slope of slow waves was calculated in the first and last hour of sleep. To account for differences in slopes due to the overnight difference in amplitude, slow waves were matched by amplitude in the first and last hour of sleep. The overnight change in the slope of slow waves reflecting sleep restoration index was calculated as (LH-FH)/FH. Therefore, a negative overnight change indicates the expected slope decline across sleep, while no negative overnight change or a positive change indicates a pathological condition.

All analyses were performed on one EEG channel which was automatically selected as the focus of the epilepsy. The focus channel was determined as the channel with the highest SWI.

Variables are described as mean and standard deviation of the mean, and median with interquartile range if not noted otherwise.

The correlation value may be a spearman's correlation coefficient.

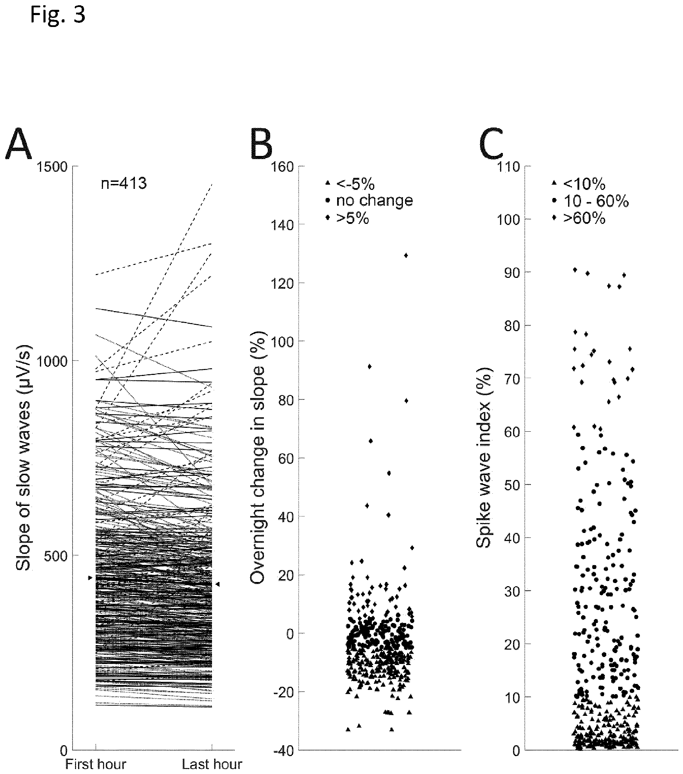

Finally, the inventors investigated sleep aspects in the patient cohort (Fig. 3A) by assessing the sleep restoration index: changes in the slope of slow waves from the first to the last hour of NREM sleep; the sleep restoration index being identical to the change of slope of slow waves in the first and last episode having the units of µV/s. In a healthy patient a decline in slope from the first to the last hour is expected. 54% of patients showed the expected overnight decrease (dotted line / triangular marker) in the slope of slow waves, but one-third (33%) of the patients showed no change (solid line / circular marker), and 13% of patients showed an overnight increase (broken line / diamond marker) (Figures 3A and 3B). The patients showing an overnight increase in the slope may represent a clinically relevant population, as their overnight change in slope is severely impaired.

Further, the spike wave index was calculated (cf. Fig. 3C), a clinically relevant marker of the severity of epilepsy. Similar to the inventor's observation regarding overnight slope changes, 52% of patients showed low rates of spike activity (triangular marker), while 42% showed high (circular marker), and 6% very high rates (diamond marker) of spike activity

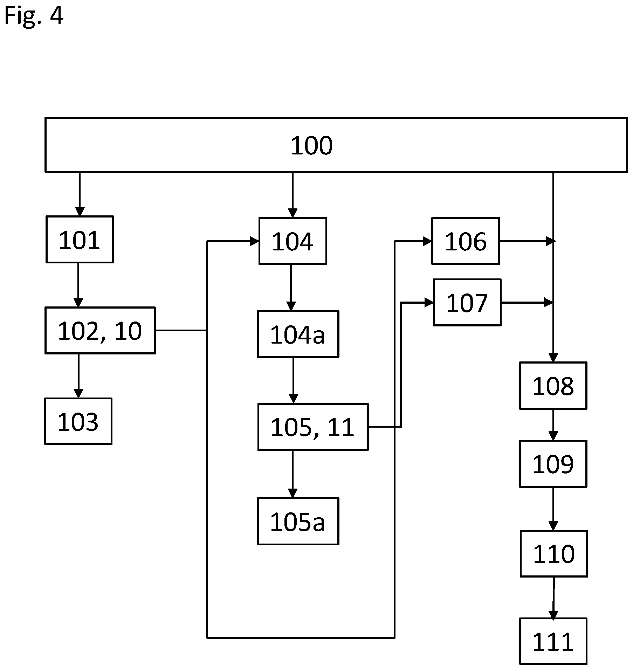

Fig. 4 shows a flow chart according to one exemplary embodiment of the invention.

In step 100, the acquired EEG data is pre-processed, comprising filtering, band-passing, down-sampling, re-referencing to contralateral mastoids.

One or more EEG channels of the EEG data are then submitted 101 as a first data set to the epileptic spike detector program module 10 that identifies and marks 102 periods of spikes in each EGG channel. For the periods of spikes the spike wave index, SWI may be calculated and displayed in step 103.

The results from the spike detector program module are then used to remove 104 the spikes from the EEG channels that are to be analyzed by the sleep detector program module 11 in form of a second data set. The EEG channels with removed spikes are than submitted to the sleep detector program module, step 105. The sleep detector program module detects and assigns 106 sleep stages in the EEG channel of non-REM sleep stage 2 and 3.

The results of the two program modules are then used 106, 107 to execute a slow-wave detection on a third data set of the EEG data. The third data set comprises filtered EEG channels, particularly EEG channels that have been bandpass filtered between 0.5 Hz to 4 Hz. On these filtered EGG channels, the slow waves are detected 108. Slow waves that are in close proximity to a detected spike, e.g. within 0.5 s, are excluded 109 from further analysis. In order to calculate the slope and the change of slope of detected slow waves, a change of slow waves with respect to a first episode of non-REM sleep stages 2 or 3, e.g. in the first hour of non-REM sleep stage 2 or 3, to a last episode of non-REM sleep stages 2 or 3, e.g. in the last hour of non-REM sleep stage 2 or 3, is determined 110. The change in the either directly assigned 111 to the sleep restoration index, or a derived entity is determined indicative of the change of slope of slow waves for the sleep restoration index.

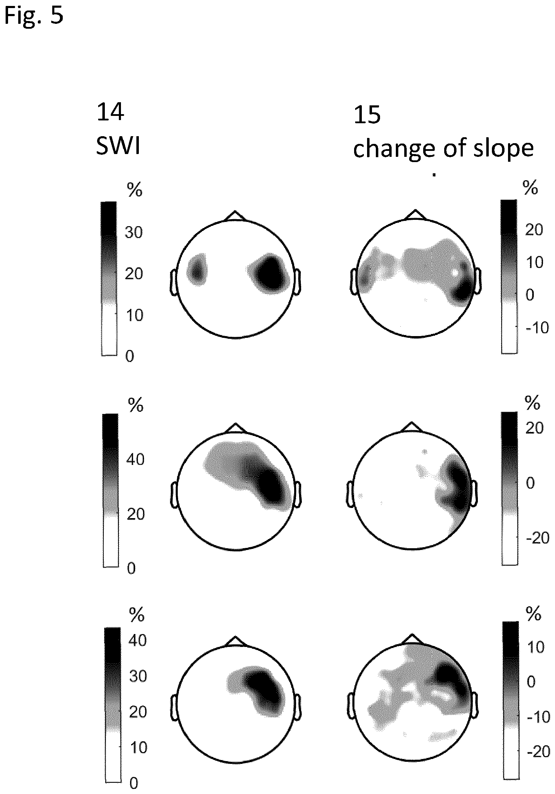

Fig. 5A) shows spike wave index map 14 and Fig. 5B) shows a change of slope map 15 determined from the same EEG data using the method according to the invention. Such maps are practically impossible to obtain by expert analysis only, as the amount of EEG data to be analysed together with the speed at which an expert is capable to analyse EEG data would require an expert to work several years on a single EEG data set. It is noted that the spike wave index map may be generated by using the invention according to the fourth independent aspect and may therefore be understood also as an independent aspect of the invention in the light of this independent method.

A clear correlation between the spike wave index and change of slope of slow waves is recognizable. There, it is possible to also calculate a correlation map, showing a spatially resolved correlation value between the spike wave index map and the change of slope map.

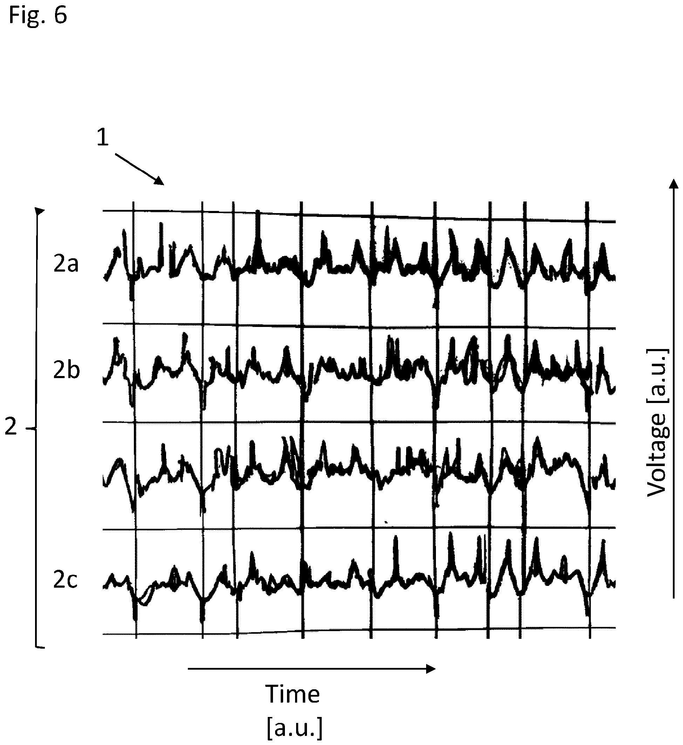

Fig. 6 shows schematically EEG data comprising a plurality of EEG channels 2, 2a, 2b, 2c recorded form different EEG sensors.

### List of reference signs:

1 EEG data
2, 2a, 2b, 2c, channels of EEG data
10 epileptic spike detector program module
11 sleep detector program module
12 slow wave detector program module
13 spike in EEG trace
14 spike wave index map
15 change of slope map
16 power spectrum
100 pre-processing of EEG-data
101 submitting EEG data to the epileptic spike detector program module
102 identification of spikes
103 determination of spike wave index
104 remove spikes from EEG data or at least some EEG channels
104 generating power spectrum
105 submit spike-free EEG data to the sleep detector program module
105a detect and assign sleep stages
106 removal of spikes in the EEG channel(s)
107 selecting slow waves form the correct sleep stage only
108 slow wave detection
109 spike-proximity exclusion of detected slow waves
110 determination of change of slope in sleep stage 2 and 3
111 determination of sleep restoration index
300 period comprising a slow wave
400 magnification of a period comprising a slow wave

## Claims

1. A computer-implemented method for determining a sleep restoration index indicative of a change of slope of slow waves during a non-REM sleep phase, the method comprising the steps of:
a. Acquiring on a computer EEG data (1) recorded from a patient, the EEG data (1) comprising information on a plurality of EEG channels (2, 2a, 2b, 2c),
b. Providing (101) a first data set comprising information on one or more EEG channels (2a) of the plurality of EEG channels (2, 2a, 2b, 2c) to an epileptic spike detector program module (10) comprising a first artificial neural network configured to detect and assign (102) periods of spikes (13) in the at least one EEG channel (2a) comprised by the first data set,
c. Providing (105) a second data set comprising information on one or more EEG channels (2b) of the plurality of EEG channels to a sleep detector program module (11) comprising a second artificial neural network configured to detect (105a) and assign sleep stages to the one or more EEG channels comprised by the second data set, wherein the detected sleep stages are selected from the group consisting of periods of non-REM sleep stages 2 and 3, and periods of other sleep stages,
d. Providing (108) a third data set comprising information on one or more EEG channels (2c) of the plurality of EEG to a slow wave detector program module (12) configured to detect and assign slow waves in the one or more EEG channels (2c) comprised by the third data set, wherein periods assigned to spikes (13) as well periods assigned to the other sleep stages are omitted for slow wave detection in the third data set,
e. Determining for at least a first episode of a period assigned to non-REM sleep stages 2 and 3, and a last episode of the period assigned to non-REM sleep stages 2 and 3 from detected slow waves, one or more slope values,
f. Determining (110) a change of slope values between the first and the last episode,
g. Determining (111) a sleep restoration index indicative for the change of the slope values.

2. The method according to claim 1, wherein from the periods of spikes (13) in the at least one EEG channel (2a) comprised by the first data set, a spike wave index is determined, wherein the spike wave index is indicative of a spike rate during the periods of spikes (13).

3. The method according to claim 2, wherein the spike detector program module (10) determines the spike wave index for a multitude of EEG channels or each EEG channel of the EEG data (1), wherein based on an associated position of each EEG channel on the head of the patient, a spike wave index map (14) of the head of the patient is generated, wherein said spike wave index map (14) comprises information on the spatial distribution of the spike wave indices.

4. The method according to claim one of the preceding claims, wherein the sleep detector program module (11) detects and assigns the sleep stage for a multitude of EEG channels or each EEG channel of the EEG data, and a change of slope values between the first and the last episode is determined for the EEG channel(s), wherein based on an associated position of each EEG channel on the head of the patient, a change of slope map (15) of the head of the patient is generated, wherein said change of slope map comprises information on the spatial distribution of the change of slope.

5. The method according to claim one of claims 2 to 4, wherein a correlation value between the change of slope and the spike wave index is determined.

6. The method according to one of the claims 1 to 5, wherein the detected spikes (13) in the periods of spikes are removed from the second data set, such that the EEG channels processed by the sleep detector program module (11) are devoid of spikes (13) detected by the spike detector program module (10) and/or wherein the detected spikes (13) in the periods of spikes are removed from the third data set, such that the EEG channels processed by the slow wave detector program module (12) are devoid of spikes (13) detected by the spike detector program module (10).

7. The method according to one of the preceding claims, wherein the second data set is processed to obtain a temporal power spectrum (16) of at least three EEG channels, wherein said power spectrum is provided to the second artificial neural network as an input feature.

8. The method according to one of the preceding claims, wherein a recording time of an electrogram comprised by the EEG data is longer than 5 minutes, particularly 0.5 hours, more particularly longer than 2 hours having a resolution of at least 128 Hz.

9. The method according to one of the preceding claims, wherein recording the EEG data and processing the EEG data according to at least the steps a. to g. is executed simultaneously.

10. The method according to claim 9, wherein the EEG data is transmitted via a data network to a cloud server comprising the computer to execute at least the steps a. to d..

11. The method according to one of the preceding claims, wherein the method is executed repeatedly on the same patient allowing for monitoring of the sleep of the patient.

12. The method according to one of the preceding claims, wherein the first artificial neural network comprises or is recurrent neural network, particularly a long short-term memory recurrent neural network and/or wherein the second artificial neural network comprises or is recurrent neural network, particularly a long short-term memory recurrent neural network.

13. The method according to one of the preceding claims, wherein at least some or all EEG channels are annotated with the detected sleep stages and with the detected periods of spikes.

14. EEG-system comprising a wearable EEG assembly with EEG sensors configured to record EEG data and a computational system connected with the wearable EEG assembly to receive EEG data recorded by the EEG assembly, wherein the computational system is configured to executed the method according to one of the preceding claims.

15. Computer program comprising computer program code that when executed on a computer or the computational system of claim 14, executes the method according to one of the claims 1 to 13.
